# EUROPEAN PATENT SPECIFICATION

(11) **EP 4 327 853 B1**
(45) Date of publication and mention of the grant of the patent: **22.04.2026**
(21) Application number: 22791567.5
(22) Date of filing: 31.03.2022
(51) Int. Cl.: A61M 25/00, A61M 25/10, A61M 25/14, A61B 18/24, A61B 18/00

(54) **ABLATION CATHETER**
ABLATIONSKATHETER
CATHÉTER D'ABLATION

(30) Priority: 23.04.2021 JP 2021073644
(43) Date of publication of application: 28.02.2024
(73) Proprietor: Nipro Corporation, Osaka-shi, Osaka 531-8510 (JP)
(72) Inventor: NISHIMURA, Yuki, Osaka-shi, Osaka 531-8510 (JP); ITO, Shuntaro, Osaka-shi, Osaka 531-8510 (JP)
(74) Representative: Slingsby Partners LLP
(86) International application number: PCT/JP2022/016442
(87) International publication number: WO 2022/224792

(56) References cited:
- WO-A1-2021/007346
- WO-A1-2021/033465
- JP-A- 2001 029 358
- JP-A- 2001 505 443
- JP-A- 2015 077 168
- JP-A- H10 504 989
- US-A- 5 620 438
- US-A1- 2018 333 588

## Description

### TECHNICAL FIELD

The present invention relates to an ablation catheter for cauterizing an inner face of a lumen such as a blood vessel.

### BACKGROUND ART

As a conventionally-known treatment, an ablation catheter is inserted into a lumen such as a blood vessel to cauterize an inner face of the lumen. For example, renal artery denervation (RDN) is a type of such treatment, which is known as a treatment to lower blood pressure of a patient with treatment-resistant hypertension by cauterizing sympathetic nerves that pass through a face of the renal artery with heat, which was conceived by focusing on the fact that the sympathetic nerves are responsible for transmitting signals to regulate the blood pressure.

As for devices for RDN, there are catheters with heaters as well as catheters for laser beam irradiation, and the applicant previously disclosed an ablation device that irradiates its target with a laser beam from the inside of a cooling balloon in Japanese Unexamined Patent Publication No. JP-A-2015-217215 (Patent Document 1).

### BACKGROUND ART DOCUMENTS

### PATENT DOCUMENTS

Patent Document 1: JP-A-2015-217215
WO 2021/033465 describes a light irradiating medical device comprising: a shaft having a first end and a second end in a longitudinal axis direction thereof and having a lumen extending in the longitudinal axis direction; a first tubular member disposed in the lumen of the shaft and rotatable about a rotation axis parallel to the longitudinal axis direction of the shaft, the first tubular member having a window located in a part of a peripheral wall of a distal portion; and a light guiding tool disposed in a lumen of the first tubular member and movable in the longitudinal axis direction, the light guiding tool including an optical fiber extending in the longitudinal axis direction, the optical fiber including a core, a cladding coating a radially outer portion of the core, and a cladding-absent portion located at a part of a distal portion of the core, and the window allowing passage therethrough of output light from the light guiding tool.

### SUMMARY OF THE INVENTION

### PROBLEM THE INVENTION ATTEMPTS TO SOLVE

In RDN, it is necessary to cauterize a plurality of locations or continuous areas in the wall of the renal artery. Therefore, it is necessary to move the optical fiber for laser beam irradiation back and forth in the length direction within the balloon that is inflated in the renal artery.

Therefore, in an ablation device for laser beam irradiation, it is desirable to improve the stability and the smoothness of movement and the operability when moving the optical fiber within the catheter, and there was still room for improvement in such areas.

The present invention has been developed in view of the above-described matters as the background, and it is an object of the present invention to provide an ablation catheter with a novel structure which is able to improve the movement of the optical fiber within the catheter in the type of ablation device that irradiates the target with a laser beam guided through the optical fiber, as shown in Patent Document 1.

### MEANS FOR SOLVING THE PROBLEM

Hereinafter, preferred embodiments for grasping the present invention will be described. However, each preferred embodiment described below is exemplary and can be appropriately combined with each other. Besides, a plurality of elements described in each preferred embodiment can be recognized and adopted as independently as possible, or can also be appropriately combined with any element described in other preferred embodiments. By so doing, in the present invention, various other preferred embodiments can be realized without being limited to those described below.

According to an aspect, there is provided an ablation catheter as claimed in claim 1.

A first preferred embodiment provides an ablation catheter comprising a reflector being disposed at a distal end side of the optical fiber so as to reflect a laser beam guided by the optical fiber toward an outer periphery for irradiation; a tubular housing member being provided to position and fix a distal end part of the optical fiber and the reflector; and a protection tube extending from a proximal end side of the housing member, the protection tube being slipped and installed externally around the optical fiber, the protection tube having a larger flexibility than the housing member.

According to this preferred embodiment, the reflector can be firmly and stably secured by the housing member at the distal end side of the optical fiber to ensure the stability of the irradiation direction with the laser beam, etc. Meanwhile, by employing the highly flexible protection tube for the optical fiber, it is possible to ensure the transmission performance of the operation force such as rotation applied to the proximal end side of the optical fiber while preventing the optical fiber from being caught in a bending part in the lumen or the like. This can improve the smoothness of movement in the length direction and the operability of the optical fiber.

A second preferred embodiment provides an ablation catheter wherein the inner tube is fixed to a distal end side of the balloon, and the inner tube is fixed to the outer tube partially in a peripheral direction, in a proximal end side of the balloon.

According to this preferred embodiment, the inner tube can be positioned in the length direction of the balloon, on both sides of the balloon in the length direction. This stabilizes the deformation state of the inner tube within the balloon, even when the balloon is bent and inflated, at a bending part in the lumen, for example, thereby preventing local large bending or slack of the inner tube. As a result, the optical fiber is prevented from getting caught in the inner tube, enabling smooth movement in the length direction and stabilizing the irradiation direction with the laser beam guided through the optical fiber. In addition, by preventing the inner tube from deviating within the balloon, the distance from the distal end of the optical fiber to the vessel wall can be kept roughly constant in the peripheral direction. For example, when the optical fiber is rotated to cauterize the sympathetic nerves around the renal artery, it is possible to cauterize substantially evenly almost over the entire periphery in the peripheral direction.

The distal end of the reinforced part of the inner tube is located in the balloon, which prevents the inner tube from collapsing even when, for example, the balloon is inflated at a bending part in the lumen and the catheter is bent significantly at the proximal end side of the balloon. This enables the catheter to maintain good mobility of the optical fiber within the inner tube.

Another preferred embodiment provides the ablation catheter wherein the reinforced part reinforced by the reinforcement in the inner tube extends to a rear end side with a predetermined length so as not to reach a proximal end of the inner tube, and a part of the inner tube from a middle in a length direction to the proximal end is the unreinforced part that does not have the reinforcement.

According to the present preferred embodiment, the flexibility of the inner tube is improved by the unreinforced part at the proximal end side of the inner tube, while maintaining the effect of preventing collapse of the inner tube caused by catheter bending, etc. at the proximal end side of the balloon as described above, thereby improving the operability for delivery into the lumen, and the like. In the present preferred embodiment, it will do as long as the unreinforced part on the proximal end side extends from the proximal end of the inner tube in the length direction to any position toward the distal end, and the "middle" does not mean the center.

Another preferred embodiment provides an ablation catheter wherein the optical fiber extends from the proximal end of the inner tube and is movable in an extraction/insertion direction; the ablation catheter further comprising a connector housing provided at a proximal end side of the catheter, a proximal end side of the outer tube and a proximal end side of the inner tube each being attached to the connector housing; and a reinforcing member being provided at a proximal end part of the optical fiber, including an area being extracted and inserted in relation to the inner tube.

According to this preferred embodiment, even when the optical fiber is moved to the end in the extraction direction (the end in the pulling direction), the reinforcing member provided at the proximal end part of the optical fiber does not completely come out from the inner tube. The reinforcing member prevents deformation of the extracted part of the optical fiber from the inner tube. Thus, for example, even when the optical fiber is subsequently operated in the insertion direction (the pushing direction), the pushing force from the proximal end of the optical fiber can be efficiently transmitted to the distal end of the optical fiber, and the pushability of the optical fiber is improved. This can improve the mobility of the optical fiber.

Another preferred embodiment provides an ablation catheter wherein a movement of the optical fiber in an axial direction in relation to the inner tube is limited to a predetermined distance.

According to this preferred embodiment, it is possible to limit unnecessary movement of the optical fiber relative to the inner tube. It also prevents, for example, the optical fiber from falling out of the inner tube, and also avoids damage due to bending or the like of the optical fiber that is drawn out to a large extent.

An example provides a usage method of the ablation catheter according to any one of the embodiments, wherein the optical fiber is moved in the axial direction in relation to the inner tube when a fluid is circulated in the balloon through the circulation passage.

Accordingly, it is possible to achieve the effects described in any of the embodiments while cooling the contact area of the balloon as described in Patent Document 1. In addition, for example, by refluxing the fluid into the balloon, deformation of the inner tube can be suppressed while maintaining the balloon in an inflated state, and smoother movement of the optical fiber can also be achieved.

### EFFECT OF THE INVENTION

According to the present invention, it is possible to improve the movement of the optical fiber within the catheter, in the ablation device of the type irradiating the target with the laser beam guided through the optical fiber as shown in Patent Document 1.

### BRIEF DESCRIPTION OF THE DRAWINGS

FIG. 1 is a vertical cross section view showing a distal end part, which is a principal part, of an ablation catheter as a first practical embodiment of the present invention.
FIG. 2 is a cross section view taken along line 2-2 of FIG. 1.
FIG. 3 is a vertical cross section view showing a proximal end part of an optical fiber unit constituting the ablation catheter shown in FIG. 1.
FIG. 4 is a vertical cross section view showing a distal end part of the optical fiber unit shown in FIG. 3.
FIG. 5 is a vertical cross section view showing a housing member constituting the distal end part of the optical fiber unit shown in FIG. 4.
FIG. 6 is a vertical cross section view showing a proximal end part of the ablation catheter shown in FIG. 1.
FIG. 7 is a vertical cross section view showing a state of the ablation catheter shown in FIG. 6 wherein the optical fiber unit is pulled to a proximal end side.
FIG. 8 is a view suitable for explaining a pre-treatment state in the ablation catheter of the present invention inserted in a vascular model.
FIG. 9 is a view suitable for explaining a state of the ablation catheter shown in FIG. 8 wherein the optical fiber unit is pulled to the proximal end side.
FIG. 10 is a view suitable for explaining a state of the ablation catheter shown in FIG. 9 wherein the optical fiber unit is pushed back to a distal end side.
FIG. 11 is a vertical cross section view showing a distal end part of an optical fiber unit constituting an ablation catheter as another practical embodiment of the present invention.
FIG. 12 is a vertical cross section view showing a housing member constituting the distal end part of the optical fiber unit shown in FIG. 11.

### EMBODIMENTS FOR CARRYING OUT THE INVENTION

Practical embodiments of the present invention will be described below in reference to the drawings.

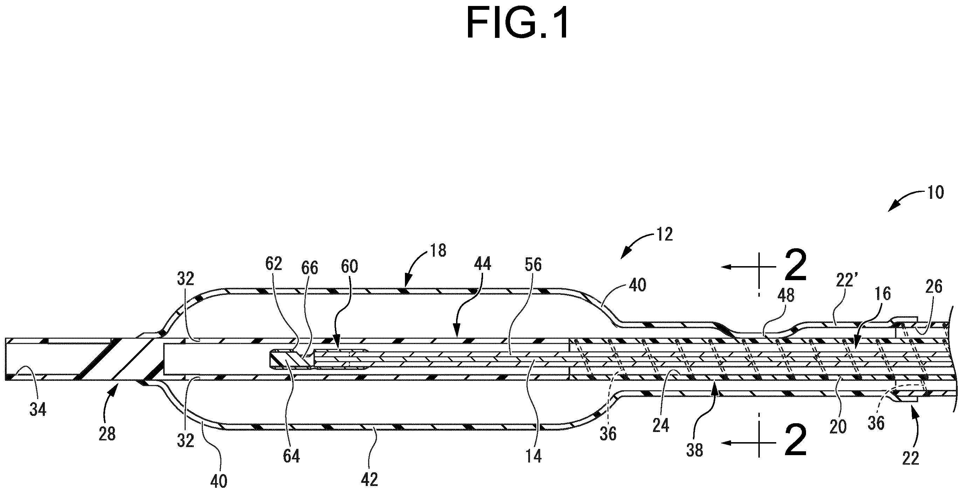

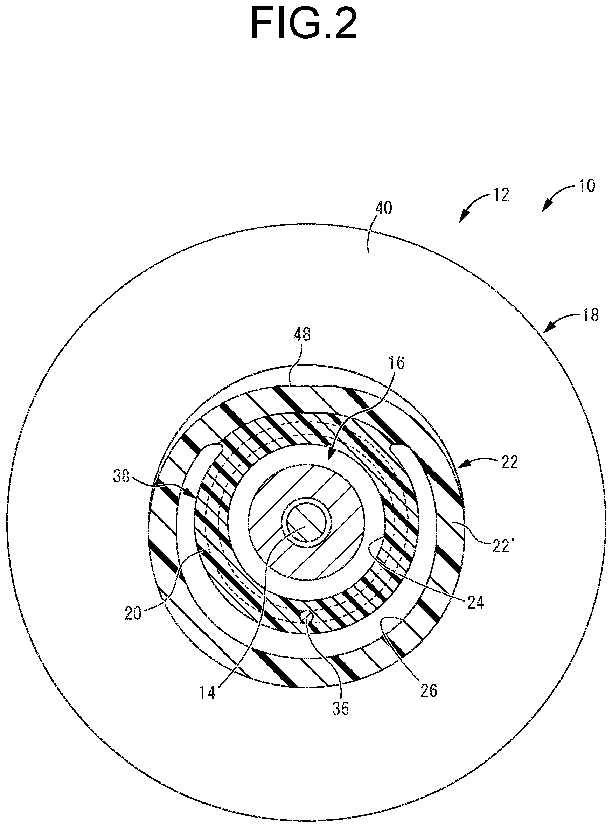

First, FIGS. 1 and 2 show a distal end part, which is a principal part of an ablation catheter 10 as a first practical embodiment of the present invention. The ablation catheter 10 has a structure in which an optical fiber unit 16 comprising an optical fiber 14 is inserted in a balloon catheter 12. This ablation catheter 10 is inserted into, for example, a renal artery, and transmits a laser beam generated from a not-shown laser beam generating means connected to the proximal end side of the ablation catheter 10, through the optical fiber 14 to the distal end side. The laser light beamed for irradiation from the distal end part of the ablation catheter 10 is to cauterize the sympathetic nerves around the renal artery. In the following explanation, the distal end side means the left side in FIG. 1, which is the side where a balloon 18 is provided in the ablation catheter 10 (the balloon catheter 12). The proximal end side means the right side in FIG. 1, which is the side where the optical fiber unit 16 is grasped and operated. In FIGS. 1 and 2, the balloon 18 is shown in an inflated state with the interior filled with a fluid such as a gas or a liquid.

In detail, the balloon catheter 12 in this practical embodiment comprises an inner tube 20, an outer tube 22 having a larger diameter than that of the inner tube 20, and the generally tubular balloon 18 provided at the distal end part of the balloon catheter 12. The inner tube 20 and the outer tube 22 each have some length dimension, and the inner tube 20 is inserted in the outer tube 22, with the inner tube 20 protruding from the distal end side of the outer tube 22. As a result, an inner lumen 24, which is the lumen of the inner tube 20, is constituted by the inner hole of the inner tube 20, and an outer lumen 26, which is the lumen of the outer tube 22, is constituted by the annular space radially between the inner tube 20 and the outer tube 22. These inner lumen 24 and outer lumen 26 each extend in the length direction of the balloon catheter 12.

A distal tip 28 is fixed to the distal end of the inner tube 20, and the inner tube 20 is integrally equipped with the distal tip 28. The distal end part of the inner tube 20, including the distal tip 28, protrudes in the length direction from the distal end of the outer tube 22.

At the distal end part of the outer tube 22, there is the balloon 18 that can expand and contract into a tubular bag shape. The balloon 18 is then disposed externally around the inner tube 20. The distal end of the balloon 18 is fixed to the outer peripheral face of the distal end of the inner tube 20 protruding from the distal end of the outer tube 22. The inner space of the balloon 18 is connected to the outer lumen 26 at the proximal end side of the balloon 18.

A through hole 32 is formed in the distal end part of the inner tube 20, through which the inner lumen 24 and the inner space of the balloon 18 are connected. The number, position, shape, and size of the through hole 32 are not limited. In the present practical embodiment, from the distal end of the inner tube 20 to some length is an unreinforced part 44 where a reinforcement in the form of a reinforcing wire 36 described below is not provided, and the through hole 32 is formed in this unreinforced part 44.

The distal tip 28 is a hollow structure, and a guidewire lumen 34 is formed in the part protruding from the balloon 18. Such guidewire lumen 34 opens to the distal end face and the outer peripheral face of the distal tip 28 to realize a rapid exchange type catheter.

For each the materials of the inner tube 20, the outer tube 22, the balloon 18, and the distal tip 28, it is possible to use synthetic resins employed in conventionally-known balloon catheters, but at least the distal end part of the inner tube 20 (the unreinforced part 44 described below) and the balloon 18 are made of a material with a high laser beam transmissivity. However, the inner tube 20 and the distal tip 28 need not be clearly distinguished, and may be integrally formed as a single component. Alternatively, the distal end of the balloon 18 may be adhered to the outer peripheral face of the distal tip 28.

The inner tube 20 of this practical embodiment has a composite structure in which the reinforcing wire 36 is embedded or adhered as the reinforcement with a higher strength than that of the tube itself. In this practical embodiment, a coiled metal wire spiraling in the axial direction (the length direction of the inner tube 20) is used as the reinforcing wire 36. This allows the inner tube 20 to be thinner than when the reinforcing wire 36 is braid-shaped, for example, as described below, and allows a wider flow path including the inner lumen 24 and the outer lumen 26. The material, structure, and the like of the reinforcing wire 36 are not limited. For example, it may be a spiral of continuous wires in the form of a band or line, a braided structure of a plurality of wires of metal or synthetic resin in a plain-woven tubular body with small or substantially no gaps, or a mesh structure of a plurality of wires in a mesh tubular shape with large gaps. In other words, the inner tube may be a braid tube.

A reinforced part 38 reinforced by the reinforcing wire 36 at the distal end side of such inner tube 20 protrudes from the distal end of the outer tube 22 and reaches into the balloon 18. It is desirable that the distal end of the reinforced part 38 reinforced by the reinforcing wire 36 should be located in a position corresponding to a site in a tapered part 40 at the proximal end side of the balloon 18 or in a straight part 42 slightly beyond the tapered part 40. This may also avoid the effect of the reinforcing wire 36 on the laser light beamed through the inner tube 20. In short, the peripheral wall part of the inner tube 20 located within the balloon 18, which the laser beam permeates, is the unreinforced part 44 provided without the reinforcing wire 36.

In the inner tube 20, the reinforced part 38 with the reinforcing wire 36 may extend to the proximal end of the inner tube 20, but in this practical embodiment, it extends from the proximal end of the balloon 18 to a predetermined length. Thus, the part of the inner tube 20 from the middle in the length direction to the proximal end includes the unreinforced part 44 that does not have the reinforcing wire 36. Specifically, for example, the proximal end position of the reinforced part 38 is set within a range of 10cm to 30cm from the proximal end of the balloon 18.

Although the structure of the inner tube 20 is not limited, the reinforced part 38 in the inner tube 20 of this practical embodiment has a three-layer structure. The inner layer is made of a highly slippery synthetic resin such as fluororesin, and the outer layer is provided sandwiching an intermediate layer that includes coiled reinforcing wires 36 with the inner layer. The material of the synthetic resin constituting the outer layer is not limited, but for example, the synthetic resin constituting the outer layer of the reinforced part 38 in the inner tube 20 is made of a more flexible material than that of the distal tip 28. This makes the reinforced part 38 in the inner tube 20 relatively flexible. That is, the reinforced part 38 of the present practical embodiment easily deforms following the bending, etc. of the blood vessel by being formed flexibly, and also has a property of being difficult to collapse because the coiled reinforcing wire 36 is embedded inside. The unreinforced part 44 of the inner tube 20 can be made of a single layer, for example, and may be formed of a different material than the synthetic resin constituting the outer layer of the reinforced part 38.

Furthermore, in this practical embodiment, a braid tube with the reinforcing wire 36 embedded in it is used for the outer tube 22, as well as the inner tube 20. The balloon 18 of this practical embodiment has a tubular part 22' integrally formed extending toward the proximal end side, and this tubular part 22' constitutes the distal end part of the outer tube 22. The lumen of the tubular part 22' is connected to the lumen of the outer tube 22 to form the outer lumen 26. The outer tube 22 and the tubular part 22' may be made of different resin materials from each other.

The inner tube 20 is inserted in the outer tube 22 and the tubular part 22' and they are partially adhered and fixed to each other in the peripheral direction, as shown in FIGS. 1 and 2. This adhered part 48 is desirably provided on the tubular part 22' at the proximal end side of the balloon 18, which does not include the reinforcing wire 36 and can be easily deformed or processed. For example, this can be achieved by applying energy such as high frequency or heating to the tubular part 22' while pressing it against the outer peripheral face of the inner tube 20 with a jig or the like to weld them. Such adhered part 48 does not extend over the entire periphery in the peripheral direction, and the specific size, shape, and position, etc. of the adhered part 48 are not limited as long as they are formed in a size that does not inhibit fluid flow in a circulation passage 82, which is described later. For example, the size of the adhered part 48 is about 1mm to 3mm in the length direction of the balloon catheter 12 and about 0.3mm to 1.5mm in the peripheral direction of the balloon catheter 12. In FIGS. 1 and 2, the thickness dimensions and the like of each part, etc. are partially exaggerated in order to show the adhered part 48 clearly.

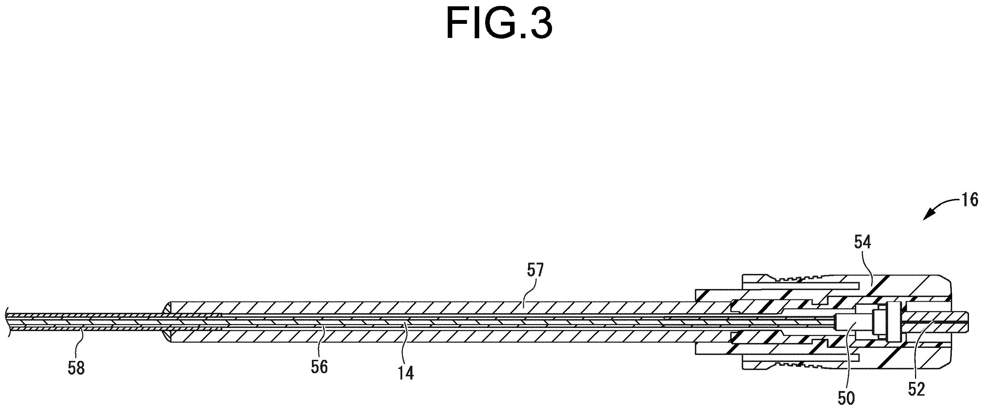

The optical fiber unit 16 inserted into the balloon catheter 12 has a fiber holding part 50 that holds the optical fiber 14, at the proximal end part thereof, as shown in FIG. 3, and the optical fiber 14 extends out from the fiber holding part 50 to the distal end side with some length. An optical fiber connector 52 protrudes from the fiber holding part 50 to the proximal end side and is connected to the optical fiber 14. By connecting the optical fiber connector 52 to the laser beam generating means, which is not shown in the figures, via an appropriate cable or the like as needed, the laser beam generated by the laser beam generating means is transmitted to the distal end side through the optical fiber 14.

As the optical fiber 14, a fiber having a refractive index of total reflection at the wavelength of the laser beam may be employed as appropriate. Specific examples are a single-mode fiber, a polarization maintaining fiber, a multi-mode fiber, a bundle fiber for image transmission, and the like. Desirably, the laser beam generated by the laser beam generating means and transmitted by the optical fiber 14 is continuous wave. The wavelength of the laser beam is preferably in the range of 400nm to 2000nm, and more preferably in the range of 800nm to 1500nm. The wavelength of the laser beam is within the above range, thus the sympathetic nerves around the renal artery can be cauterized more stably, for example.

The proximal end part of the optical fiber unit 16 has a fixing part 54 fixed to a connector housing 70 described below, and the fixing part 54 is fixed to the fiber holding part 50. This fixing part 54 has, for example, an approximately tubular shape, and is fixed to the fiber holding part 50 so as to cover the outer peripheral side of the fiber holding part 50. This allows the optical fiber unit 16 to be stably assembled to the connector housing 70 by gripping the fixing part 54.

Here, a protection tube 56 is attached to the optical fiber 14 as the protection tube 56 is slipped externally around the optical fiber 14. In this practical embodiment, the protection tube 56 is provided over substantially the entire length of the optical fiber 14. In this practical embodiment, the protection tube 56 is constituted by a metal coil, and in particular, in this practical embodiment, the protection tube 56 is constituted by a stainless steel (SUS) wire wrapped around the optical fiber 14. The protection tube 56 is fixed to the optical fiber 14 by bonding the distal end part and the proximal end part to the optical fiber 14. By providing the spiral-shaped protection tube 56, the rotational torque exerted on the proximal end part can be stably transmitted to the distal end side of the optical fiber unit 16 when the optical fiber unit 16 is rotated and operated at the hand side (the proximal end side) of the ablation catheter 10 during the cautery treatment described below.

Furthermore, the proximal end part of the optical fiber 14 is provided with a tubular protection tube member 57 that covers and protects the optical fiber 14 from the outside, and the protection tube member 57 extends out from the fixing part 54 to the distal end side. Furthermore, a reinforcing member 58 is provided at the proximal end part of the optical fiber 14. In this practical embodiment, the reinforcing member 58 is constituted by a metal pipe having a certain length, and in particular, in this practical embodiment, the pipe is made of stainless steel (SUS). The reinforcing member 58 is disposed around the optical fiber 14 and the protection tube 56, and extends out from, for example, the protection tube member 57 to the distal end side. The reinforcing member 58 is shorter in length than the optical fiber 14 and the protection tube 56, and the distal end of the protection tube member 57 is bonded to the outer peripheral face of the reinforcing member 58. The reinforcing member need not be made of metal, but may be made of a hard synthetic resin, for example. The reinforcing member need not be tubular, but may be partially provided in the peripheral direction, for example, with a C-shaped cross-section.

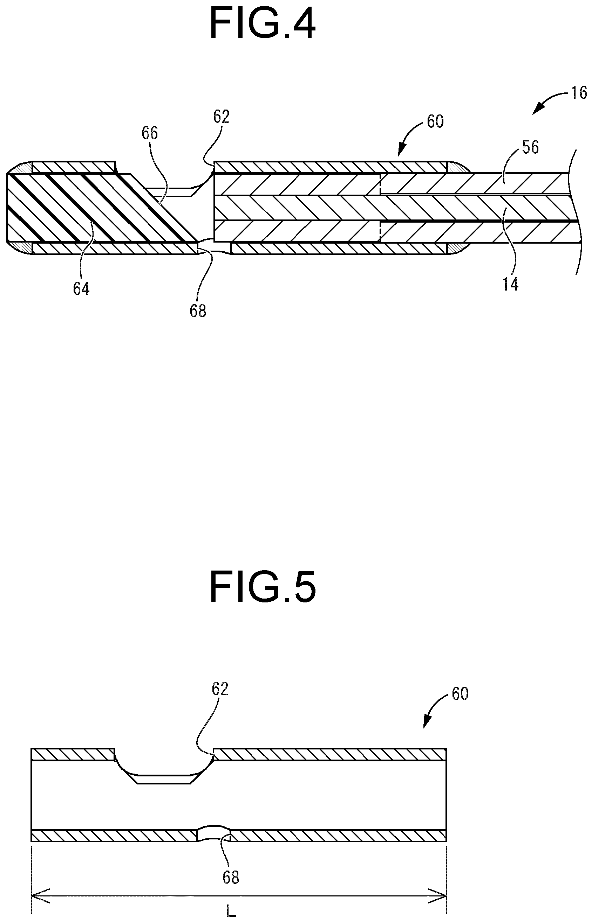

On the other hand, a roughly tubular housing member 60 is provided at the distal end part of the optical fiber unit 16, as shown in FIGS. 4 and 5. In the middle part in the length direction of the peripheral wall of the housing member 60, a through window 62, which passes through the housing member 60 in the thickness direction, is provided partially in the peripheral direction and opens laterally in the length (axial) direction of the optical fiber unit 16 (the ablation catheter 10). In the housing member 60, the optical fiber 14 around which the protection tube 56 is disposed is fixed at the proximal end side of the through window 62. A reflector 64 is fixed at the distal end side of the through window 62 to reflect the laser beam transmitted through the optical fiber 14 to the distal end side. The proximal end face of the reflector 64 is an inclined face that is inclined relative to the axial direction and is a reflective face 66 that reflects the laser beam. The distal end face of the optical fiber 14 and the reflective face 66 face each other at some distance in the axial direction.

The housing member 60 is a rigid member formed of a metal or a synthetic resin, for example, and has a greater deformation rigidity than that of the protection tube 56, which is fixed to the housing member 60. In other words, the protection tube 56 has a larger flexibility than the housing member 60. The reflector 64 may be formed of a synthetic resin and have a metallic film or coating on the reflective face 66, for example, or the entire reflector 64 may be formed of a metal.

The optical fiber 14 with the protection tube 56 disposed around it is inserted into the housing member 60 from the proximal end side. That is, the protection tube 56 extends from the proximal end side of the housing member 60. Furthermore, the reflector 64 is inserted into the housing member 60 from the distal end side, and the distal end part of the protection tube 56 and the reflector 64 are fixed to the housing member 60. In addition, a positioning hole 68 is provided in the peripheral wall of the housing member 60, opposite the through window 62 in the diametrical direction, so as to serve as a marker when the reflector 64 is assembled to the housing member 60.

In this practical embodiment, the through window 62 and the positioning hole 68 are provided in positions where they partially overlap each other in the axial direction. The positioning hole 68 below is visible through the through window 62 in a plan view of the housing member 60 (viewed from above in FIGS. 4 and 5). In this practical embodiment, the optical fiber 14 about which the protection tube 56 is disposed is inserted from the proximal end side of the housing member 60 to a position where it is not exposed through the through window 62 when viewed in the plan view. The reflector 64 is inserted from the distal end side of the housing member 60 to a position where the positioning hole 68 is not covered when viewed in the plan view. As a result, the distal end part of the optical fiber 14 and the reflector 64 are positioned and fixed to the housing member 60.

In particular, in this practical embodiment, the through window 62 is provided on the distal end side relative to the center of the housing member 60 in the length direction. This allows a sufficiently long adhesion allowance between the protection tube 56, which is disposed about the optical fiber 14, and the housing member 60. The length dimension L (see FIG. 5) of the housing member 60 is not limited, but is suitably 3.0mm or less, and more suitably 2.5mm or less. In this practical embodiment, the length dimension L of the housing member 60 is 2.5mm. By setting the length dimension L of the housing member 60 within the above numerical range, the deformation rigidity of the distal end part of the optical fiber unit 16 can be kept small, and the distal end part of the optical fiber unit 16 can be easily deformed to follow the bending part of a lumen such as a blood vessel into which the ablation catheter 10 is inserted.

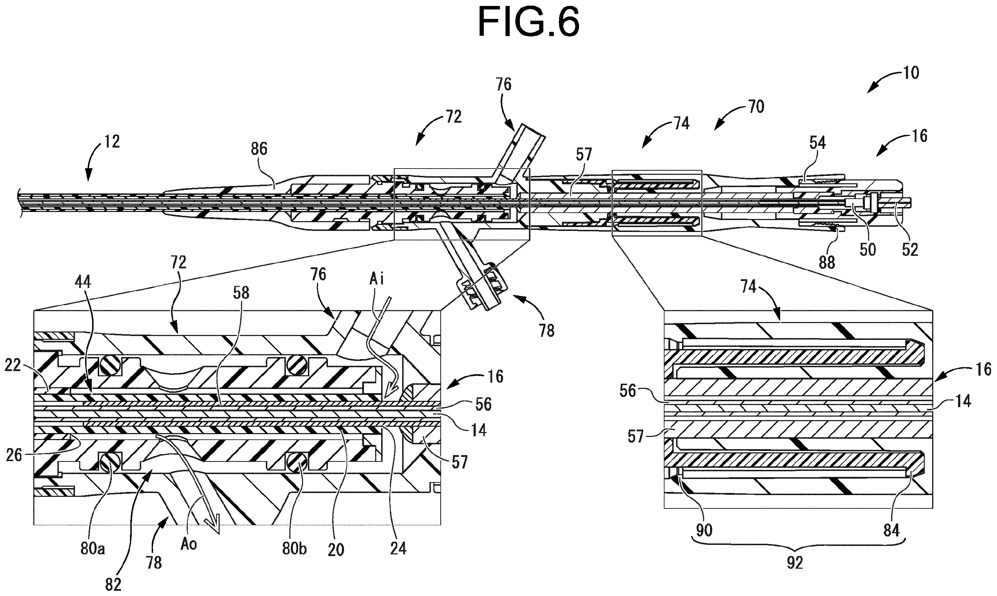

As shown in FIG. 6, the ablation catheter 10 of this practical embodiment is equipped with the connector housing 70 at the proximal end that can be grasped and operated during use. The connector housing 70 has a generally tubular shape as a whole and is constituted by a plurality of synthetic resin components assembled together. In particular, in this practical embodiment, the connector housing 70 includes a roughly tubular distal end side part 72 constituted by a plurality of members and an approximately tubular proximal end side part 74 constituted by a plurality of members. The distal end side part 72 and the proximal end side part 74 are assembled in the length direction (the axial direction) of the connector housing 70, in a mutually movable manner.

The balloon catheter 12 is fixedly attached to the distal end side part 72 of the connector housing 70, and the optical fiber unit 16 is fixedly attached to the proximal end side part 74. The optical fiber unit 16 extending from the proximal end side part 74 to the distal end side is inserted into the inner lumen 24, which is an inner hole of the inner tube 20 in the balloon catheter 12, and the distal end part of the optical fiber unit 16 (the housing member 60) is located at the distal end part of the inner lumen 24. That is, the optical fiber unit 16 including the optical fiber 14 is inserted in the inner tube 20 in a movable manner. The optical fiber 14 extending from the proximal end of the inner tube 20 is movable in the extraction/insertion direction as the optical fiber unit 16 is pulled and pushed as described below.

The peripheral wall of the distal end side part 72 of the connector housing 70 is provided with an inner side port 76 and an outer side port 78, which protrude externally through in the thickness direction. In other words, the inner space of the distal end side part 72 is connected to the outer space through the inner side port 76 and the outer side port 78. As a result, a fluid such as a gas or a liquid can flow into the inner space of the distal end side part 72 through the inner side port 76 and the outer side port 78. The proximal end parts of the inner tube 20 and the outer tube 22 of the balloon catheter 12 are inserted into the distal end side part 72 from the distal end side, and the proximal end sides of the inner tube 20 and the outer tube 22 and the distal end side part 72 are fixed to each other.

The inner side port 76 is located on the proximal end side of the outer side port 78, and the inner tube 20 extends to the proximal end side further than the outer tube 22. The inner lumen 24, which is the inner hole of the inner tube 20, is connected to the inner side port 76, and the outer lumen 26 between the inner tube 20 and the outer tube 22 is connected to the outer side port 78. In FIG. 6, the thickness dimensions of the components are exaggerated for clarity, and it is difficult to distinguish the inner lumen 24 and the outer lumen 26, but both the inner lumen 24 and the outer lumen 26 open to the proximal end side in the inner space of the distal end side part 72.

Here, a pair of O-rings 80a, 80b are provided on the distal end side part 72, axially spaced apart from each other, and the inner side port 76 is connected to the inner lumen 24 at the proximal end side of the proximal O-ring 80b, and the outer side port 78 is connected to the outer lumen 26 axially between the pair of O-rings 80a, 80b. This prevents the fluid flowing from the inner side port 76 and the fluid flowing from the outer side port 78 from mixing within the distal end side part 72.

That is, the fluid that flows into the distal end side part 72 from the inner side port 76, as shown by arrow Ai in FIG. 6, flows into the balloon 18 through the through holes 32, 32 of the inner lumen 24 and a lumen 30. The fluid that flows into the balloon 18 is discharged from the outer side port 78 through the outer lumen 26 as shown by the arrow Ao in FIG. 6. This allows the supply of fluid to the balloon 18 and the discharge of fluid from the balloon 18, and the circulation passage 82 is configured to extend from the inner side port 76 through the balloon 18 to the outer side port 78. The fluid flowing in the circulation passage 82 is not limited, but it is possible to employ a mixture of physiological saline and contrast medium, for example. For fluid flow in the circulation passage 82, for example, a pump, etc., not shown in the figure, provided outside the ablation catheter 10 may be used.

In this practical embodiment, as shown in the enlarged view on the right side in FIG. 6, an outer peripheral claw part 84 is provided at the proximal end of the distal end side part 72, which protrudes to the outer peripheral side. This outer peripheral claw part 84 may be provided over the entirety in the peripheral direction or partially in the peripheral direction. Furthermore, an approximately tubular kink prevention cover 86 is attached to the distal end of the distal end side part 72 to prevent kinking of the balloon catheter 12 that is inserted into the kink prevention cover 86.

On the other hand, at the proximal end of the proximal end side part 74, there is a retaining part 88 that engages with the fixing part 54 of the optical fiber unit 16 to hold the proximal end side part 74 and the optical fiber unit 16 in a fixed state. By engaging these fixing part 54 and retaining part 88, the proximal end side part 74 and the optical fiber unit 16 in the connector housing 70 can be operated integrally. The distal end part of the proximal end side part 74 is formed in a size that can cover the proximal end part of the distal end side part 72, and when the distal end side part 72 and the proximal end side part 74 are assembled, the distal end side part 72 and the proximal end side part 74 are partially overlapped in the axial direction, in a state one is inside the other in the radial direction.

The protection tube member 57 and the reinforcing member 58 of the optical fiber unit 16 inserted into the proximal end side part 74 are inserted from the proximal end side into the distal end side part 72. In this practical embodiment, in the initial state before treatment (the state shown in FIG. 6), the distal end of the reinforcing member 58 is inserted from the proximal end side into the inner lumen 24 of the inner tube 20 and located in an axial position approximately equal to the proximal end of the outer tube 22. Although it is difficult to discern in FIG. 6, the outer diameter dimension of the reinforcing member 58 is smaller than the inner diameter dimension of the inner tube 20, and the inner lumen 24 is open at the proximal end side on the outer peripheral side of the reinforcing member 58.

In this practical embodiment, as shown in the enlarged view on the right side in FIG. 6, an inner peripheral claw part 90 is provided at the distal end part of the proximal end side part 74, so as to protrude to the inner peripheral side. The inner peripheral claw part 90 may be provided over the entirety in the peripheral direction or partially in the peripheral direction, at the location corresponding to the outer peripheral claw part 84. The proximal part of the distal end side part 72 is inserted into the distal end part of the proximal end side part 74, and the outer peripheral claw part 84 goes beyond the inner peripheral claw part 90, thereby assembling the proximal end side part 74 to the distal end side part 72 in an axially movable and rotatable manner around the central axis. Furthermore, when the optical fiber unit 16 is pulled, as described below, the outer peripheral claw part 84 and the inner peripheral claw part 90 contact each other to limit the amount of pulling operation of the optical fiber unit 16. A stopper mechanism 92 is constituted including the outer peripheral claw part 84 and the inner peripheral claw part 90 so as to limit the amount of pulling operation of the optical fiber unit 16.

There will be explained a specific example of the usage method of the ablation catheter 10 of the present practical embodiment. First, the ablation catheter 10 of this practical embodiment is delivered to the treatment site (the cauterization site) following the guide wire inserted into the renal artery in advance. After confirming that the ablation catheter 10 has been delivered to the treatment site under the X-ray fluoroscopy, for example, the fluid is allowed to flow into the ablation catheter 10 through the inner side port 76. This supplies the fluid to the inside of the balloon 18 through the inner lumen 24 and the through holes 32, 32 to dilate the balloon 18. The balloon 18 is then expanded and pressed against the inner wall of the renal artery to secure the balloon 18 to the treatment site in the renal artery. The fluid supplied to the inside of the balloon 18 is discharged through the outer lumen 26 from the outer side port 78. The fluid discharged from the outer side port 78 may be allowed to flow back into the balloon 18 through the inner side port 76 again, or may be pooled outside the ablation catheter 10.

With the balloon 18 secured to the treatment site in the renal artery and the fluid circulated in the balloon 18 through the circulation passage 82, the laser beam generating means is activated to transmit the laser beam through the optical fiber 14 toward the distal end side and the ablation catheter 10 irradiates the target from the distal end face of the optical fiber 14. The laser light beamed from the optical fiber 14 is reflected on the reflective face 66 in the reflector 64 so as to irradiate the side, which is the outer periphery of the ablation catheter 10, through the through window 62 provided on the side of the optical fiber unit 16. Such laser beam irradiates the inner wall of the renal artery through the unreinforced part 44 of the inner lumen 24 and the balloon 18, which are made transparent to cauterize the sympathetic nerves located outside the blood vessel by heating the blood vessel from the inside.

Here, by pulling the proximal end side part 74 toward the proximal end side in relation to the distal end side part 72 of the connector housing 70, the optical fiber unit 16 fixed to the proximal end side part 74 can be moved integrally toward the proximal end side in relation to the balloon 18 fixed to the renal artery, thus moving the laser beam cauterization position toward the proximal end side. The irradiation direction of the laser beam can be changed in the peripheral direction by rotating the proximal end side part 74 with respect to the distal end side part 72 around the central axis. The operation of moving the proximal end side part 74 to the proximal end side relative to the distal end side part 72 and the operation of rotating the proximal end side part 74 can be performed separately, but can also be performed simultaneously to cauterize the sympathetic nerves around the renal artery in a spiral shape. In particular, by performing the cautery treatment by moving the optical fiber 14 in the axial direction in relation to the inner tube 20 while the fluid is circulated in the balloon 18 through the circulation passage 82, the sympathetic nerves around the renal artery can be cauterized while cooling the part in the renal artery that contacts the balloon 18. In addition, by maintaining the balloon 18 pressurized by returning the fluid into the balloon 18 with a pump, etc., for example, the balloon 18 is more stably fixed to the renal artery and deformation of the balloon 18 is suppressed. This, combined with suppression of deformation of the inner tube 20 within the balloon 18, allows the optical fiber 14 to move within the inner tube 20 more smoothly.

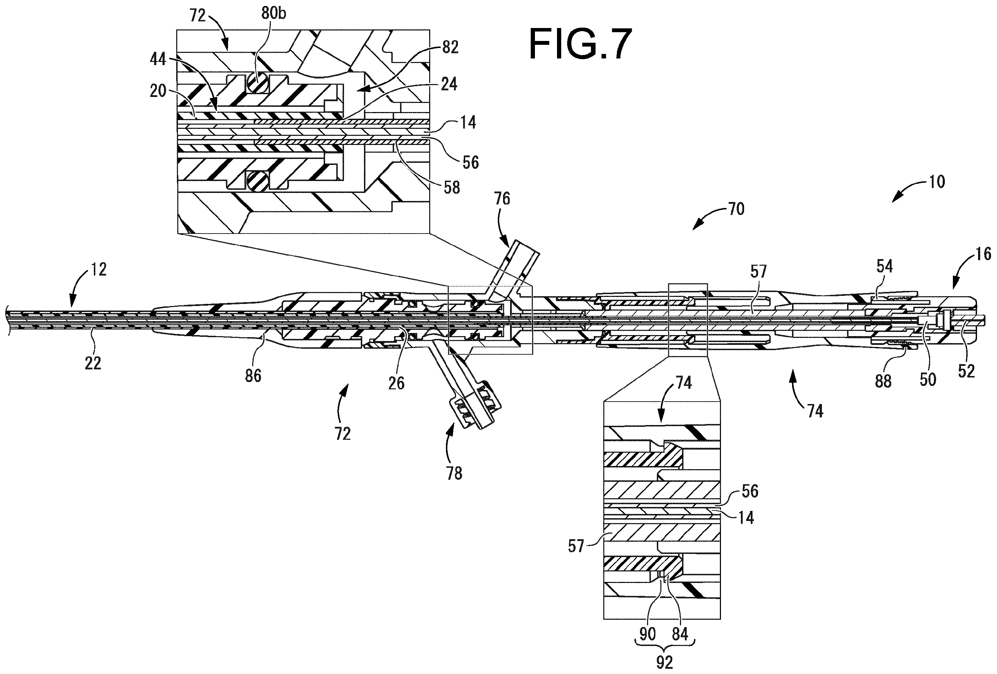

When changing the cauterization position to the proximal end side by moving the proximal end side part 74 to the proximal end side relative to the distal end side part 72 in this way, as shown in the enlarged right side view in FIG. 7, the outer peripheral claw part 84 and the inner peripheral claw part 90 contact each other. This limits the amount of pulling operation of the proximal end side part 74 relative to the distal end side part 72, namely, the amount of movement of the optical fiber unit 16 toward the proximal end relative to the balloon catheter 12. In short, the movement of the optical fiber 14 in the axial direction in relation to the inner tube 20 is limited to a predetermined distance. This suppresses unnecessary movement of the optical fiber unit 16 (the optical fiber 14) and prevents the optical fiber unit 16 from being extracted out of the balloon catheter 12 to the proximal end side.

In particular, in this practical embodiment, the reinforcing member 58 provided in the optical fiber unit 16 is prevented from slipping out of the inner tube 20 in the state where the outer peripheral claw part 84 and the inner peripheral claw part 90 contact each other to limit the movement of the proximal end side part 74 to the proximal end side, as shown in the enlarged left side view in FIG. 7. In summary, the distance of movement of the proximal end side part 74 toward the proximal end relative to the distal end side part 72 allowed by the stopper mechanism 92 is smaller than the length dimension of the reinforcing member 58 that has entered the inner tube 20 in the initial state shown in FIG. 6. That is, in this practical embodiment, the reinforcing member 58 is provided at the proximal end part of the optical fiber 14, including the area which is extracted and inserted in relation to the inner tube 20. As a result, even during the pulling operation of the proximal end side part 74 shown in FIG. 7, the reinforcement state of the optical fiber 14 by the reinforcing member 58 is stably maintained between the inner tube 20 and the protection tube member 57 in the axial direction. For example, when the proximal end side part 74 is pulled toward the proximal end and the proximal end side part 74 (the optical fiber unit 16) is then pushed again toward the distal end, the pushing force toward the distal end side can be transmitted stably to the distal end of the optical fiber unit 16 without breaking the optical fiber 14.

As shown in FIG. 7, when the proximal end side part 74 is moved toward the proximal end relative to the distal end side part 72 and the outer peripheral claw part 84 and the inner peripheral claw part 90 are in contact with each other, the protection tube member 57 of the optical fiber unit 16 is not extracted out from the distal end side part 72. This prevents the optical fiber 14 from being damaged even when the optical fiber unit 16 is pulled to the proximal end side, as the proximal end side part 74 is tilted relative to the distal end side part 72, or the optical fiber unit 16 is pulled out from the balloon catheter 12, or the like.

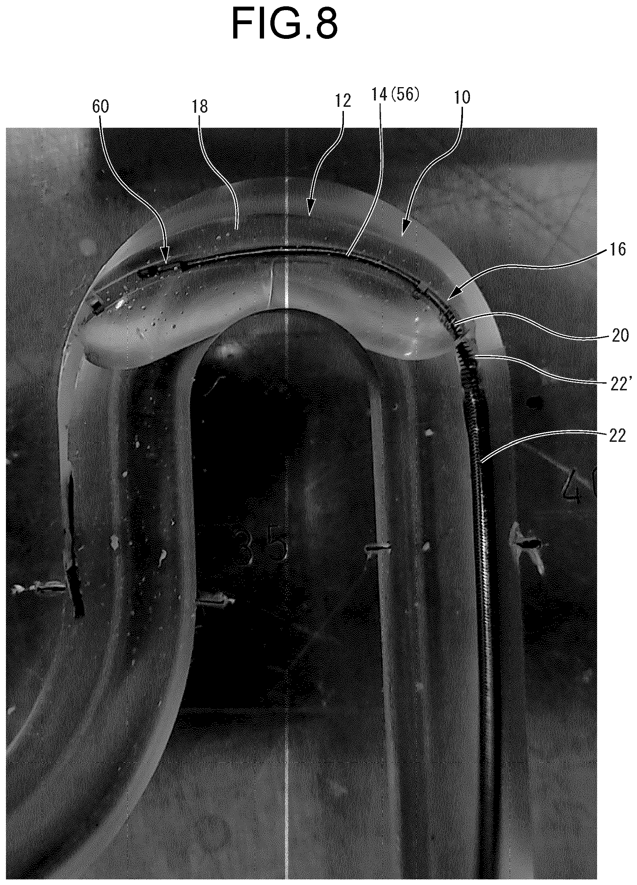

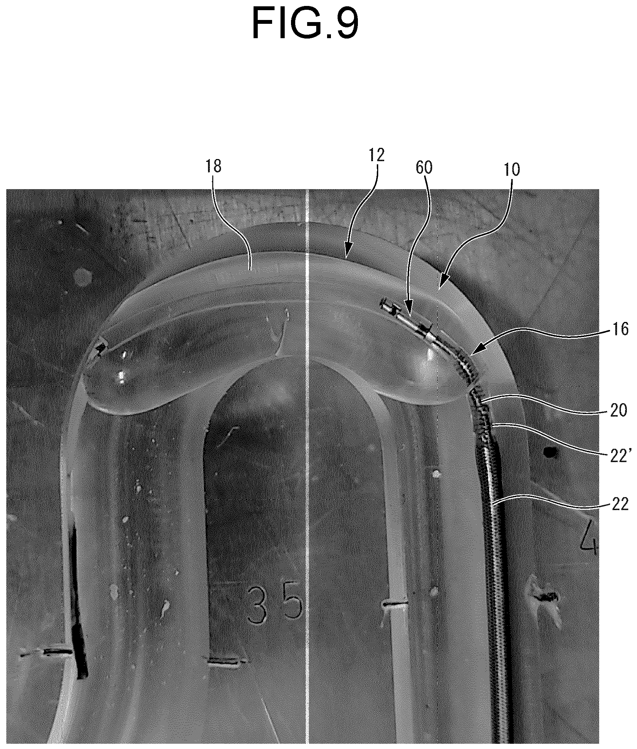

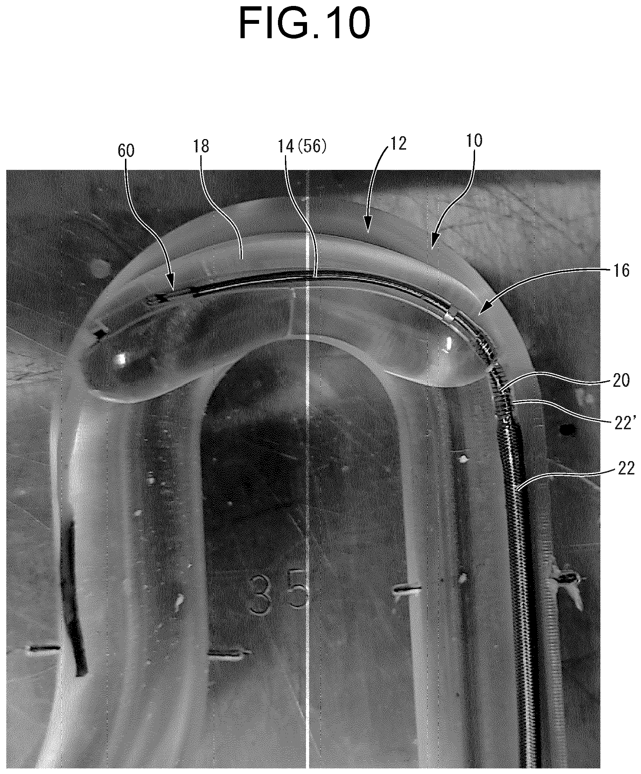

The applicant actually confirmed that the optical fiber unit 16 moves well in the axial direction relative to the balloon catheter 12 using a vascular model, by making a prototype of the ablation catheter 10 of this practical embodiment. The results are shown in FIGS. 8 to 10. FIG. 8 shows the initial state before treatment, FIG. 9 shows the state in which the optical fiber unit 16 is pulled toward the proximal end, and FIG. 10 shows the state in which the optical fiber unit 16 is pushed toward the distal end again after the state in FIG. 9. These results show that the optical fiber unit 16 moves relative to the balloon catheter 12 without getting caught even when the blood vessel bends extremely as shown in FIGS. 8 to 10, and that the tensile and pushing forces by the connector housing 70 are stably transmitted to the distal end of the optical fiber unit 16. In particular, by comparing FIG. 8 and FIG. 10, it was confirmed that even when the optical fiber unit 16 was pulled to the proximal end side and then pushed in again, it returned to substantially the same position as the initial state before treatment.

According to the ablation catheter 10 of this practical embodiment with the structure described above, the protection tube 56 extending from the housing member 60 to the proximal end side in the optical fiber unit 16 has a greater flexibility than the housing member 60. As a result, in the optical fiber unit 16, it can deform following the bending of the lumen of a blood vessel or the like without becoming rigid as a whole, and the mobility of the optical fiber unit 16 can be improved. In particular, by setting a small length dimension L of the housing member 60, the distal end part of the optical fiber unit 16 can be more easily deformed, and thus the mobility of the optical fiber unit 16 can be further improved.

In the ablation catheter 10 of the present practical embodiment, the inner tube 20 and the outer tube 22 are partially adhered to each other in the peripheral direction at the proximal end side of the balloon 18. This prevents, for example, displacement or deformation of the inner tube 20 within the inflated balloon 18, and thus prevents the movement of the optical fiber unit 16 from being obstructed by the deformed wall, etc. of the inner tube 20. In other words, the optical fiber unit 16 can move well within the inner tube 20, and the mobility is improved.

Furthermore, in this practical embodiment, the reinforcing wire 36 as the reinforcement is embedded in the inner tube 20 to prevent the inner tube 20 from collapsing. This stably keeps the shape of the inner lumen 24 into which the optical fiber unit 16 is inserted even in a bending lumen, for example, thereby improving the mobility of the optical fiber unit 16 within the inner lumen 24. The distal end of the inner tube 20 (the reinforced part 38) reaches the inside of the balloon 18. As a result, the distal end part of the optical fiber unit 16 can more reliably reach the inside of the balloon 18 and thus the desired cauterization position.

Furthermore, in this practical embodiment, the reinforcing member 58 is provided at the proximal end part of the optical fiber 14, including an area to be inserted and extracted in relation to the inner tube 20. That is, in the initial state, the reinforcing member 58 is inserted in the inner tube 20, and the reinforcing member 58 is not extracted out of the inner tube 20 even when the optical fiber unit 16 is moved to the proximal end side. This keeps the reinforcement state of the optical fiber 14 pulled out from the inner tube 20, by the reinforcing member 58. The optical fiber 14 does not bend when the optical fiber unit 16 is pushed in again, for example, and the pushing force can be transmitted stably to the distal end. Therefore, by providing the reinforcing member 58, the pushability of the optical fiber unit 16 can be improved and the mobility can be enhanced. Particularly, the stopper mechanism 92 is provided in this practical embodiment, which not only prevents the optical fiber 14 from falling out or being damaged due to extraction, but also more securely maintains the reinforced state of the optical fiber 14 by the reinforcing member 58.

Although the practical embodiments of the invention have been described in detail above, the present invention is not limited by that specific description.

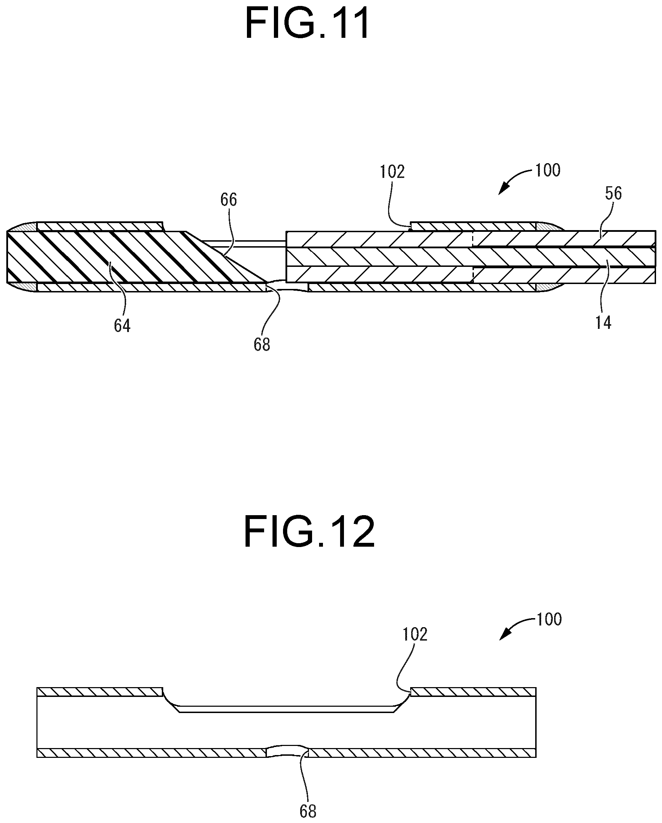

For example, the housing member that can be employed in the present invention is not limited to those shown in FIGS. 4 and 5, etc., but a housing member 100 shown in FIGS. 11 and 12 can also be employed. In the present embodiment, a through window 102 is provided at the center part in the length direction of the housing member 100, and the length of the part to which the reflector 64 is fixed and the length of the part to which the optical fiber 14 (the protection tube 56) is fixed in the housing member 100 are approximately equal. The distal end of the optical fiber 14 (the protection tube 56) is positioned so that the positioning hole 68 is partially covered from the proximal end side. In FIGS. 11 and 12, members and parts that are substantially the same as those in the previous practical embodiment are marked with the same symbols as those in the previous practical embodiment in the figures to omit detailed description. When the housing member 100 is in such a shape, the same effects as in the aforesaid practical embodiment can be achieved.

In the above-mentioned practical embodiment, the balloon catheter 12 constituting the ablation catheter 10 was a rapid exchange type. However, for example, a lumen other than the lumen through which the optical fiber unit is inserted, may be provided along the entire length of the balloon catheter in the length direction and used as a guidewire lumen. In other words, the balloon catheter may be an over-the-wire type.

### KEYS TO SYMBOLS

10 ablation catheter
12 balloon catheter
14 optical fiber
16 optical fiber unit
18 balloon
20 inner tube
22 outer tube
22' tubular part
24 inner lumen
26 outer lumen
28 distal tip
32 through hole
34 guidewire lumen
36 reinforcing wire (reinforcement)
38 reinforced part
40 tapered part
42 straight part
44 unreinforced part
48 adhered part
50 fiber holding part
52 optical fiber connector
54 fixing part
56 protection tube
57 protection tube member
58 reinforcing member
60 housing member
62 through window
64 reflector
66 reflecting face
68 positioning hole
70 connector housing
72 distal end side part
74 proximal end side part
76 inner side port
78 outer side port
80a, 80b O-ring
82 circulation passage
84 outer peripheral claw part
86 kink prevention cover
88 retaining part
90 inner peripheral claw part
92 stopper mechanism
100 housing member
102 through window

## Claims

1. An ablation catheter (10) comprising:
an outer tube (22);
an inner tube (20) being inserted in the outer tube (22);
a balloon (18) provided at a distal end side of the outer tube (22);
a fluid circulation passage (82) being formed by a lumen (24) of the inner tube (20) and a lumen (26) of the outer tube (22) being connected in the balloon (18); and
an optical fiber (14) being inserted in the inner tube (20) in a movable manner, wherein
the inner tube (20) is a tube including a reinforcement (36),
in a distal end side of the inner tube (20), an unreinforced part (44) is provided without the reinforcement (36) so that a laser beam guided by the optical fiber (14) permeates the unreinforced part (44),
the ablation catheter (10) being **characterized in that**
a distal end of a reinforced part (38) reinforced by the reinforcement (36) in the inner tube (20) is positioned in the balloon (18).

2. The ablation catheter (10) according to claim 1, wherein the reinforced part (38) reinforced by the reinforcement (36) in the inner tube (20) extends to a rear end side with a predetermined length so as not to reach a proximal end of the inner tube (20), and a part of the inner tube (20) from a middle in a length direction to the proximal end is the unreinforced part (44) that does not have the reinforcement (36).

3. The ablation catheter (10) according to claim 1 or 2, further comprising:
a reflector (64) being disposed at a distal end side of the optical fiber (14) so as to reflect the laser beam guided by the optical fiber (14) toward an outer periphery for irradiation;
a tubular housing member (60, 100) being provided to position and fix a distal end part of the optical fiber (14) and the reflector (64); and
a protection tube (56) extending from a proximal end side of the housing member (60, 100), the protection tube (56) being slipped and installed externally around the optical fiber (14), the protection tube (56) having a larger flexibility than the housing member (60, 100).

4. The ablation catheter (10) according to any one of claims 1-3, wherein:
the inner tube (20) is fixed to a distal end side of the balloon (18), and
the inner tube (20) is fixed to the outer tube (22) partially in a peripheral direction, in a proximal end side of the balloon (18).

5. The ablation catheter (10) according to any one of claims 1-4, wherein:
the optical fiber (14) extends from the proximal end of the inner tube (20) and is movable in an extraction/insertion direction;
the ablation catheter (10) further comprising:
a connector housing (70) provided at a proximal end side of the catheter (10), a proximal end side of the outer tube (22) and a proximal end side of the inner tube (20) each being attached to the connector housing (70); and
a reinforcing member (58) being provided at a proximal end part of the optical fiber (14), including an area being extracted and inserted in relation to the inner tube (20).

6. The ablation catheter (10) according to any one of claims 1-5 wherein:
a movement of the optical fiber (14) in an axial direction in relation to the inner tube (20) is limited to a predetermined distance.

## Patentansprüche

1. Ablationskatheter (10), umfassend:
ein äußeres Rohr (22);
ein inneres Rohr (20), das in das äußere Rohr (22) eingeführt ist;
einen Ballon (18), der an einer distalen Endseite des äußeren Rohrs (22) bereitgestellt ist;
einen Fluidzirkulationskanal (82), der dadurch ausgebildet ist, dass ein Lumen (24) des inneren Rohrs (20) und ein Lumen (26) des äußeren Rohrs (22) in dem Ballon (18) verbunden sind; und
eine optische Faser (14), die auf eine bewegliche Weise in das innere Rohr (20) eingeführt ist, wobei
das innere Rohr (20) ein Rohr ist, das eine Verstärkung (36) enthält,
in einer distalen Endseite des inneren Rohrs (20) ein unverstärkter Teil (44) ohne die Verstärkung (36) bereitgestellt ist, sodass ein durch die optische Faser (14) geführter Laserstrahl den unverstärkten Teil (44) durchdringt,
wobei der Ablationskatheter (10) **dadurch gekennzeichnet ist, dass**
ein distales Ende eines verstärkten Teils (38), der durch die Verstärkung (36) in dem inneren Rohr (20) verstärkt ist, in dem Ballon (18) positioniert ist.

2. Ablationskatheter (10) nach Anspruch 1, wobei sich der verstärkte Teil (38), der durch die Verstärkung (36) in dem inneren Rohr (20) verstärkt ist, zu einer hinteren Endseite mit einer vorbestimmten Länge so erstreckt, dass er ein proximales Ende des inneren Rohrs (20) nicht erreicht, und ein Teil des inneren Rohrs (20) von einer Mitte in einer Längsrichtung zu dem proximalen Ende der unverstärkte Teil (44) ist, der die Verstärkung (36) nicht aufweist.

3. Ablationskatheter (10) nach Anspruch 1 oder 2, ferner umfassend:
einen Reflektor (64), der an einer distalen Endseite der optischen Faser (14) angeordnet ist, um den durch die optische Faser (14) geführten Laserstrahl zur Bestrahlung zu einem äußeren Umfang hin zu reflektieren;
ein rohrförmiges Gehäuseelement (60, 100), das bereitgestellt ist, um einen distalen Endteil der optischen Faser (14) und den Reflektor (64) zu positionieren und zu fixieren; und
ein Schutzrohr (56), das sich von einer proximalen Endseite des Gehäuseelements (60, 100) erstreckt, wobei das Schutzrohr (56) außen um die optische Faser (14) gesteckt und installiert ist,
wobei das Schutzrohr (56) eine größere Flexibilität als das Gehäuseelement (60, 100) aufweist.

4. Ablationskatheter (10) nach einem der Ansprüche 1-3, wobei:
das innere Rohr (20) an einer distalen Endseite des Ballons (18) fixiert ist und
das innere Rohr (20) an dem äußeren Rohr (22) teilweise in einer Umfangsrichtung in einer proximalen Endseite des Ballons (18) fixiert ist.

5. Ablationskatheter (10) nach einem der Ansprüche 1-4, wobei:
sich die optische Faser (14) von dem proximalen Ende des inneren Rohrs (20) erstreckt und in eine Auszieh-/Einführrichtung beweglich ist;
der Ablationskatheter (10) ferner Folgendes umfasst:
ein Verbindergehäuse (70), das an einer proximalen Endseite des Katheters (10) bereitgestellt ist, wobei eine proximale Endseite des äußeren Rohrs (22) und eine proximale Endseite des inneren Rohrs (20) jeweils an dem Verbindergehäuse (70) angebracht sind; und
ein Verstärkungselement (58), das an einem proximalen Endteil der optischen Faser (14) bereitgestellt ist und einen Bereich enthält, der in Bezug auf das innere Rohr (20) herausgezogen und eingeführt wird.

6. Ablationskatheter (10) nach einem der Ansprüche 1-5, wobei:
eine Bewegung der optischen Faser (14) in einer axialen Richtung in Bezug auf das innere Rohr (20) auf einen vorbestimmten Abstand begrenzt ist.

## Revendications

1. Cathéter d'ablation (10) comprenant :
un tube externe (22) ;
un tube interne (20) inséré dans le tube externe (22) ;
un ballonnet (18) prévu au niveau d'un côté d'extrémité distale du tube externe (22) ;
un passage de circulation de fluide (82) formé par une lumière (24) du tube interne (20) et une lumière (26) du tube externe (22) connectées dans le ballonnet (18) ; et une fibre optique (14) insérée dans le tube interne (20) de manière mobile,
ledit tube interne (20) étant un tube comprenant un renfort (36),
dans un côté d'extrémité distale du tube interne (20), une partie non renforcée (44) étant prévue sans le renfort (36) afin qu'un faisceau laser guidé par la fibre optique (14) se diffuse dans la partie non renforcée (44), le cathéter d'ablation (10) étant **caractérisé en ce que**
une extrémité distale d'une partie renforcée (38) renforcée par le renfort (36) dans le tube interne (20) est positionnée dans le ballonnet (18).

2. Cathéter d'ablation (10) selon la revendication 1, ladite partie renforcée (38) renforcée par le renfort (36) dans le tube interne (20) s'étendant vers un côté d'extrémité arrière avec une longueur prédéfinie de façon à ne pas atteindre une extrémité proximale du tube interne (20), et une partie du tube interne (20) à partir d'un milieu dans une direction de longueur vers l'extrémité proximale étant la partie non renforcée (44) qui ne comporte pas le renfort (36).

3. Cathéter d'ablation (10) selon la revendication 1 ou 2, comprenant en outre :
un réflecteur (64) disposé au niveau d'un côté d'extrémité distale de la fibre optique (14) de façon à réfléchir le faisceau laser guidé par la fibre optique (14) vers une périphérie externe pour l'irradiation ;
un élément de logement tubulaire (60, 100) prévu pour positionner et fixer une partie d'extrémité distale de la fibre optique (14) et du réflecteur (64) ; et
un tube de protection (56) s'étendant à partir d'un côté d'extrémité proximale de l'élément boîtier (60, 100), le tube de protection (56) étant glissé et installé à l'extérieur autour de la fibre optique (14), le tube de protection (56) possédant une plus grande flexibilité que l'élément boîtier (60, 100).

4. Cathéter d'ablation (10) selon l'une quelconque des revendications 1 à 3, ledit tube interne (20) étant fixé à un côté d'extrémité distale du ballonnet (18) et ledit tube interne (20) étant fixé partiellement au tube externe (22) dans une direction périphérique, dans un côté d'extrémité proximale du ballonnet (18).

5. Cathéter d'ablation (10) selon l'une quelconque des revendications 1 à 4,
ladite fibre optique (14) s'étendant à partir de l'extrémité proximale du tube interne (20) et étant mobile dans une direction d'extraction/insertion ;
ledit cathéter d'ablation (10) comprenant en outre :
un boîtier connecteur (70) prévu au niveau d'un côté d'extrémité proximale du cathéter (10), un côté d'extrémité proximale du tube externe (22) et un côté d'extrémité proximale du tube interne (20), chacun étant fixé au boîtier connecteur (70) ; et
un élément de renforcement (58) prévu au niveau d'une partie d'extrémité proximale de la fibre optique (14), comprenant une zone extraite et insérée par rapport au tube interne (20).

6. Cathéter d'ablation (10) selon l'une quelconque des revendications 1 à 5,
un mouvement de la fibre optique (14) dans une direction axiale par rapport au tube interne (20) étant limité à une distance prédéfinie.
